# EUROPÄISCHE PATENTSCHRIFT

(11) **EP 1 470 790 B1**
(45) Veröffentlichungstag und Bekanntmachung des Hinweises auf die Patenterteilung: **05.04.2006**
(21) Anmeldenummer: 03009326.4
(22) Anmeldetag: 24.04.2003
(51) Int. Cl.: A61B 17/58, A61B 17/88

(54) **Instrumentensystem für Pedikelschrauben**
Instrumentsystem for pedicle screw
Ensemble d'instruments pour vis pédiculaire

(43) Veröffentlichungstag der Anmeldung: 27.10.2004
(73) Patentinhaber: Zimmer GmbH, 8404 Winterthur (CH)
(72) Erfinder: Casutt, Simon, 9200 Gossau (CH); Meier, Nimrod, 8200 Schaffhausen (CH); Huber, Marc, 8488 Turbenthal (CH)
(74) Vertreter: Manitz, Finsterwald & Partner GbR

(56) Entgegenhaltungen:
- DE-A- 10 005 134
- FR-A- 2 703 288
- US-B1- 6 235 028
- US-B1- 6 251 112

## Beschreibung

Die vorliegende Erfindung handelt von einem Instrumentensystem mit den Merkmalen des Oberbegriffs des Anspruchs 1. Pedikelschrauben mit Klemmschrauben in ihrem Kopf sind in der Patentschrift EP-A-0669 109 beschrieben. Die Firma Centerpulse Orthopedics Ltd, CH-6340 Baar, Schweiz vertreibt unter dem Namen DYNESYS ® ein Stabilisationssystem mit Pedikelschrauben und zugehörigen Instrumenten für Rückenwirbel. Bei dem Anbringen von Implantaten an der Wirbelsäule war es bisher nur eingeschränkt möglich wenig invasive Zugangswege zu benutzen, wenn die natürlichen Strukturen weitgehend erhalten bleiben sollen (Approach Prof. Wiltse transmuskulär, oder Paraspinal Approach). Das Operationsfeld musste zum Teil von den mittig liegenden Dornfortsätzen her freipräpariert werden. Das Setzen von Pedikelschrauben und das daran anschliessende Verbinden mit versteifenden Elementen setzt bei dem Operateur grosse Erfahrung und Fertigkeit voraus. Des Weiteren wird zum Stand der Technik auf DE 100 05 134 A1 verwiesen.

Aufgabe der vorliegenden Erfindung ist es, diesen Zustand zu verbessern und dem Operateur mehr Sicherheit zu geben. Diese Aufgabe wird gemäss dem kennzeichnenden Teil des unabhängigen Anspruchs 1 gelöst.

Diese Anordnung hat den Vorteil, dass eine Madenschraube als Klemmschraube ausserhalb vom eigentlichen Operationsfeld in den rohrförmigen Teil einsetzbar ist und unter ihrem Eigengewicht oder durch den Drehschrauber gestossen an den Kopf der Pedikelschraube gleitet und dort einschraubbar ist, ohne dass ein die Madenschraube greifendes Instrument notwendig ist und ohne Risiko, dass die Madenschraube unbeabsichtigt an einem anderen Ort im Operationsfeld abgesetzt wird. Dadurch, dass der Innendurchmesser D₂ des rohrförmigen Teils nur wenig grösser als der Aussendurchmesser D₁ der Madenschraube ist, behält diese die Lage in der sie in den rohrförmigen Teil eingesetzt wurde. Der Drehschrauber findet seinen Formschluss beispielsweise in einem Innensechkant in der Madenschraube und die Madenschraube findet das Gewinde im Kopf der Pedikelschraube. Ein sicheres "sich finden" der Gewinde wird erreicht, wenn der Innendurchmesser D₂ weniger als das 1,3-fache des Aussendurchmessers D₁ der Madenschraube beträgt.

Einen wesentlichen Vorteil bringt ein statt der Klemmschraube zwischenzeitlich durch das rohrförmige Teil einsetzbares Zentrierteil, welches ebenfalls im Gewinde des Kopfes einschraubbar ist und über das rohrförmige Teil in Längsrichtung nach aussen vorsteht.

Ein so verankertes Zentrierteil erlaubt es, den Halter abzuziehen und den gleichen oder andere Halter geführt, und ohne direkte Sicht auf die Pedikelschraube, wieder aufzusetzen. Durch diese Massnahme kann die Pedikelschraube ohne Madenschraube durch einen ersten Halter gesetzt werden, der ein entsprechendes rohrförmiges Teil aufweist, in welches statt der Madenschraube ein Zentrierteil einsetzbar ist, welches an seinem vorstehenden Ende greifbar ist, um es an dem Kopf der Pedikelschraube festzuschrauben. Dabei ist der grösste Durchmesser D des Zentrierteils im Anschluss an einen kurzen Gewindeteil geringfügig grösser als der Gewindedurchmesser um eine Abstützschulter zu bilden, aber immer noch kleiner als der Innendurchmesser D₂ des rohrförmigen Teils. Ein so verankertes Zentrierteil gestattet es einen ersten Halter vom Kopf der Pedikelschraube abzuziehen und andere Halter mit anderen Funktionen ohne direkte Sicht auf den Kopf der Pedikelschraube beliebig oft aufzusetzen. So kann zum Beispiel ein erster Halter als kräftiges Eindrehwerkzeug konzipiert werden, welches sich formschlüssig auf späteren Funktionsflächen des Kopfes abstützt und kann nach dem Eindrehen des Zentrierteils zu einem späteren Zeitpunkt auf einen anderen Halter gewechselt werden, der seinerseits aufgeschoben wird, bis er fomschlüssig an Hilfsflächen des Kopfes anliegt, um nach dem Abziehen des Zentrierteils das Einbringen und Festsetzen der Madenschraube zu ermöglichen. Dieser neu aufgesetzte Halter führt dann Madenschraube und Drehschrauber und ermöglicht gleichzeitig ein Gegenmoment beim Festsetzen der Madenschraube.

Die abhängigen Ansprüche stellen vorteilhafte Weiterbildungen der Erfin-dung dar. Das Zentrierteil ermöglicht also den Wechsel von Haltern mit verschiedenen Funk-tionen, ohne dass der Kontakt zum Kopf der Pedikelschraube verloren geht.

Günstiger als ein starr ausgeführtes Zentrierteil ist die erfindungsgemäße biegeelastische Ausführung, die durch ihre Nachgiebigkeit verhindert, dass trotz der grossen Hebellänge des aufgeschraubten Zentrierteils, bei dessen Auslenkung grosse Kräfte an der Pedikelschraube entstehen. Zudem lassen sich die Zentrierteile soweit wegbiegen, dass andere Geräte z.B. Bildwandler/Röntgengeräte einsetzbar sind.

Wenn man erfindungsgemäß den in Längsrichtung mittleren Bereich elastisch ausführt, so dass im mittleren Bereich eine Winkelabweichung von beispielsweise 20 ° von der Längsachse möglich ist, dann kann ein am Zentrierteil geführter Halter auch in einem Bogen an den Kopf einer unsichtbaren Pedikelschraube herangefürt werden und das abdeckende Gewebe schonend verdrängen. Eine Möglichkeit die notwendige Elastizität:im mittleren Bereich zu erzeugen besteht darin, einen biegeelastischen Werkstoff für den mittleren Teil zu verwenden und/oder den Querschnitt so zu verringern, dass keine unzulässigen Biegespannungen entstehen.

Eine weitere Möglichkeit besteht darin, den mittleren Teil als mehrfachen Draht oder als Schraubenfeder auszuführen, um eine notwendige Elastizität zu erreichen.

Um die Führung des Zentrierteils beim Aufsetzen eines Halters zu vereinfachen ist es vorteilhaft, wenn das Zentrierteil kreisförmige Querschnitte und Mantellinien mit sanften stufenlosen Übergängen aufweist.

Wenn man einen ersten rohrförmigen Halter mit einer Klemmvorrichtung versieht, welche ein in der Pedikelschraube eingeschraubtes Zentrierteil in axialer Richtung beispielsweise durch Klemmen blockiert, kann diese Kombination als Setzinstrument für die Pedikelschraube verwendet werden. Man setzt ausserhalb vom menschlichen Körper das Zentrierteil auf die Pedikelschraube auf und sichert die Position eines nachträglich aufgesetzten Halters in axialer Richtung indem man diesen gegenüber dem Zentrierteil verspannt, um anschliessend mit dem Halter die Pedikelschraube einzudrehen. Nach dem Lösen der Klemmung, kann der als Setzinstrument verwendete Halter, weil beispielsweise die formschlüssig an ihm anliegenden Flächen der Pedikelschraube anderweitig benutzt werden sollen, abgezogen werden und ein anderer Halter kann entlang dem Zentrierteil an den Kopf der Pedikelschraube herangeführt werden. Ein solcher anderer Halter kann beispielsweise ein Halter mit einem rohrförmigen Teil sein, welches am Ende zur Pedikelschraube als Umlenkvorrichtung einen vorstehenden Schuh mit einem Umlenkbogen für ein durch den Schraubenkopf als Verbindungsteil hindurch gezogenes Band aufweist und am anderen Ende des rohrförmigen Teils - also ausserhalb des eigentlichen Operationsfeldes- bezüglich Drehung des rohrförmigen Teils eine formschlüssige Kupplungsfläche für einen Bandspanner aufweist, welche den Bandspanner in Richtung zu der Pedikelschraube abstützt. Diese Anordnung ermöglicht ein Vorspannen ausserhalb des eigentlichen Operationsfeldes und kommt somit mit geringeren Abmessungen für die Zugangswege aus. Bandspanner die für eine solche Anwendung tauglich sind, werden in der Patentschrift EP-A-1103225 beschrieben. Da solche Bänder, die vor ihrer Fixierung durch die Klemmschraube mit dem Bandspanner auf eine vorgegebene Vorspannkraft gebracht werden, auch eine gewisse Biegesteifigkeit aufweisen, ist es vorteilhaft den Umlenkbogen mit einem Radius grösser 3 mm zu gestalten. Auf diese Weise entsteht durch die Umlenkung kein zu grosses Biegemoment am durchgezogenen Band im Durchgang des Schraubenkopfes und somit auch keine zu grosse Reibung, die das Messergebnis der angelegten Vorspannung massgeblich verfälschen könnte.

Für Pedikelschrauben des oben erwähnten Produktes DYNESYS ® kann mit dem vorgängig beschriebenen Instrumentensystem beispielsweise folgende Operationsfolge beim Setzen in zwei benachbarten Wirbeln vorgenommen werden, ohne dass der Kontakt zur jeweiligen Pedikelschraube verloren geht:

### 1. Pedikelschraube

a1) Eindrehen und Verankern eines Zentrierteils auf dem Kopf der Pedikelschraube ausserhalb des Operationsfeldes. Aufsetzen eines ersten Halters bis er formschlüssig auf dem Kopf anliegt und sichern -besser noch vorspannen- des Halters am Zentrierteil in axialer Richtung. Eindrehen der Pedikelschraube in den Wirbelkörper.
b1) Lösen der axialen Sicherung und Abziehen des Halters; das Zentrierteil bleibt stehen.
c1) Einziehen eines Bandes durch den Schraubenkopf der Pedikelschraube mit hier nicht beschriebenen zangenähnlichen Instrumenten, wobei eine Orientierung und eventuelle Führung an dem Zentrierteil möglich ist.
d1) Aufsetzen eines zweiten Halters mit rohrförmigern Teil entlang dem Zentrierteil, wobei der zweite Halter in zwei seitlichen Kerben des Schraubenkopfes formschlüssig anliegt.
e1) Entfernen des Zentrierteils durch Lösen der Verschraubung und durch Abziehen und stattdessen zunächst eine Madenschraube und anschliessend einen Drehschrauber in den rohrförmigen Teil einführen, um das Band durch das Eindrehen der Klemmschraube endgültig zu befestigen. Der Drehschrauber kann dafür eine Anzeige des Drehmomentes mit entsprechender Messeinrichtung aufweisen. Abziehen des Drehschraubers.

### 2. Pedikelschraube

a2) Analog zu a1) Aufsetzen des ersten Halters ...
b2) Analog zu b1) d. h. ein Zentrierteil bleibt in der eingedrehten Pedikelschraube stehen.

### Zwischenschritte:

- Bestimmen des Abstandes der 1. und 2. Pedikelschraube unter einer vorgegebenen Spreizkraft, um die notwendige Länge für ein auf das Band aufschiebbares Kissen festzulegen. (Für diese Einrichtung ist eine Parallelanmeldung mit dem gleichen Anmeldedatum eingereicht). Das Spreizinstrument kann sich für das Ansetzen an dem eingedrehten Zentrierteil der zweiten Pedikelschraube und am Band an der ersten Pedikelschraube orientieren.
- Herstellen der vorgegebenen Kissenlänge durch Abschneiden vom Überstand und Aufziehen des Druckkissens auf das Band, bis das Kissen an der 1. Pedikelschraube anschlägt.

### 2. Pedikelschraube

c2) Analog zu c1) Einziehen des Bandes.
d2) Analog zu d1) Aufsetzen eines weiteren Halters, mit rohrförmigern Teil, der jedoch zusätzlich einen vorstehenden Schuh mit Umlenkbogen und am anderen Ende d.h. ausserhalb des eigentlichen Operationsfeldes eine formschlüssige Aufnahme für einen Bandspanner aufweist.
e2) Mit dem aufgesetzten Bandspanner soviel Vorspannung anbringen, bis das Kissen an beiden Pedikelschrauben mit seinen vorgesehenen Kontaktflächen anliegt. Das Zentrierteil entfernen und stattdessen eine Madenschraube und den Drehschrauber durch den rohrförmigen Teil einführen; mit dem Bandspanner eine vorgesehene Vorspannkraft erzeugen und die Madenschraube bei dieser Vorspannkraft am Band mit dem Drehschrauber unter einem vorgegebenen Drehmoment anziehen, um das Band zu sichern, wobei mit den Bandspannern über die formschlüssige Aufnahme das Gegenmoment gleichzeitig aufgenommen werden kann.

Falls eine weitere Pedikelschraube an einem anschliessenden Wirbel vorgesehen ist, kann wie für die 2. Pedikelschraube vorgegangen werden. Grundsätzlich können auch als erstes alle Pedikelschrauben mit dem ersten Halter gesetzt werden. Um so wichtiger ist dann, dass die Zentrierteile jeweils in den Pedikelschrauben angeschraubt verbleiben. Dabei ist für jede weitere einzusetzende Pedikelschraube deren Lage an den bereits herausragenden Zentrierelementen leichter abschätzbar.

Dadurch dass bei diesem Instrumentensystem die Halter einen rohrförmigen Teil aufweisen, welcher mit seinem Innendurchmesser auf die Ausrichtung der Madenschraube abgestimmt ist und vorstehende Zentrierteile statt der Madenschrauben durch die Halter hindurch in den Köpfen der Pedikelschrauben befestigbar sind, kann der Kontakt zur eingesetzten Pedikelschraube auch bei nicht einsehbarem d.h. kleinerem Operationsfeld aufrecht erhalten werden.

Im folgenden wird die Erfindung anhand von Ausführungsbeispielen beschrieben. Es zeigen:
- Fig.1:: Schematisch zwei benachbarte Wirbel, in denen entsprechend der oben erwähnten EP-A-0669101 Pedikelschrauben eingedreht sind, welche über ein Band und ein Druckkissen unter Vorspannung miteinander verbunden sind;
- Fig.2:: Entspricht einer Draufsicht auf den Schraubenkopf einer Pedikelschraube von Figur 1;
- Fig.3:: Schematisch den unteren Teil von einem ersten erfindungsgemässen Halter, welcher mit einem Zentrierteil ein Eindrehwerkzeug für eine Pedikelschraube bildet;
- Fig.4:: Schematisch einen oberen Teil zu der Anordnung von Figur 3 in welchem eine axiale Sicherung des Zentrierteils im Halter gezeigt ist;
- Fig.5:: Schematisch ein weiteres Zentrierteil, welches im Kopf einer Pedikeslchraube eingeschraubt ist;
- Fig.6:: Schematsich einen zweiten Halter, der mit Vorsprüngen in Kerben am Kopf einer Pedikelschraube eingreift;
- Fig.7:: Den unteren Teil vom Halter in Figur 6 um 90 ° gedreht im Schnitt;
- Fig.8:: Eine Klemmschraube, die als Madenschraube ausgeführt ist;
- Fig.9:: Einen Drehschrauber für eine Madenschraube nach Fig.8;
- Fig.10:: Schematisch ein Spreizinstrument, mit dem der Abstand von zwei Pedikelschrauben unter einer vorgegebenen Vorspannkraft bestimmt wird.
- Fig.11:: Schematisch einen Halter mit einem rohrförmigen Teil, an den ein Umlenkschuh angesetzt ist.
- Fig.12:: Schematisch eine Seitenansicht des Halters von Figur 12 der auf den Kopf einer Pedikelschraube aufgesetzt ist;
- Fig.13:: Schematisch den Halter von Figur 12 um 90 ° gedreht im Schnitt;
- Fig. 14:: Schematisch den unteren Teil von einem ersten Halter analog zu Figur 3, der jedoch zu einem Instrumentensatz für eine Klemmschraube gehört, welche als Sechskantschraube ausgebildet ist;
- Fig. 15:: Schematisch im Schnitt den unteren Teil von einem zweiten Halter im Instrumentensatz zu Figur 14 mit einer Sechskantschraube, die von einem Drehschrauber gehalten wird und ein Band im Kopf einer Pedikelschraube festsetzt;
- Fig.16:: Schematisch eine um 90 ° gedrehte Seitenansicht auf den Drehschrauber von Figur 15;
- Fig.17:: Schematisch eine stirnseitige Ansicht auf Drehschrauber und Schraube von Figur 15;
- Fig. 18 und 19:: Schematisch eine weitere Ausführung von einem Zentrierteil und einem ersten Halter, welche als Eindrehwerkzeug analog zu Figuren 3 und 4 kombiniert sind;
- Fig. 20:: Schematisch und vergrössert eine von Hand betätigbare Spannschraube aus Figur 19;
- Fig.21:: Schematisch eine vergrösserte Ansicht der Stimfläche der Spannschraube aus Figur 20, an der die Kontur des Kopfes sichtbar ist;
- Fig.22:: Schematisch, sehr stark vergrössert den Kopf einer Pedikelschraube gemäss Figur 2. welcher eine kreisringförmige, ebene Abstützfläche in axialer Richtung aufweist; und
- Fig.23: Schematisch den unteren Teil von einem Halter analog zu Figur 11, der jedoch statt einem Umlenkschuh eine Umlenkrolle aufweist.

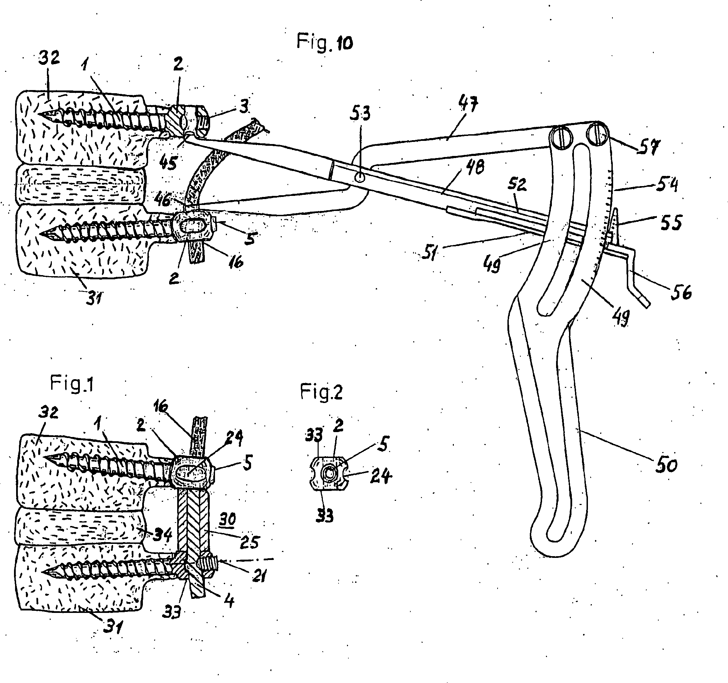

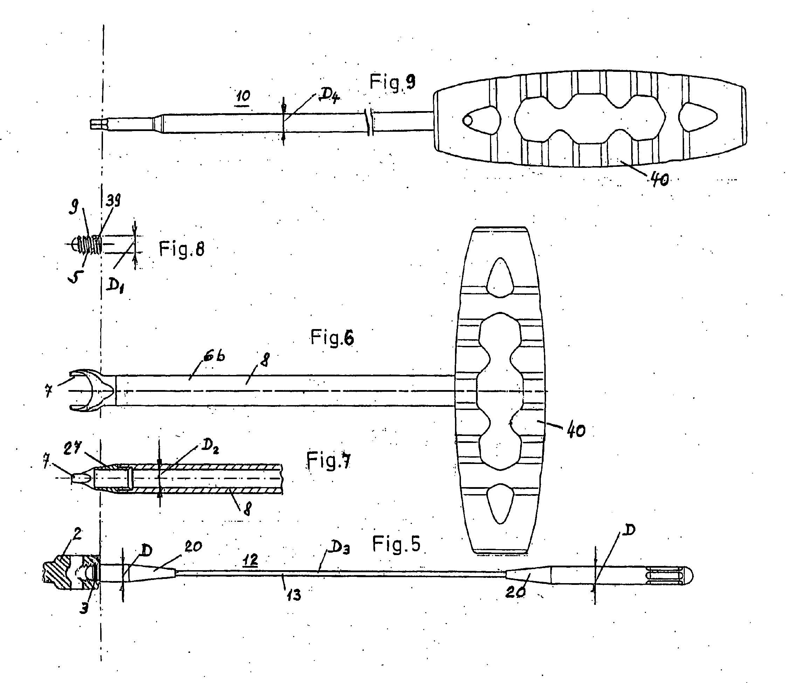

In den Figuren 1 und 2 ist ein fertig montiertes Stützelement 30 zwischen zwei benachbarten Rückenwirbeln 31, 32 gezeigt, welches mit einem erfindungsgemässen Instrumentensystem montierbar ist. Die beiden Pedikelschrauben 1 besitzen einen Kopf 2, der kugelförmig ist und jeweils auf zwei gegenüberliegenden Seiten abgeflachte Stirnflächen 33 aufweist, welche Abstützflächen für ein zylindrisches Druckkissen 25 bilden. Quer zu den Stirnflächen 33 ist in einer Bohrung ein elastisches, vorgespanntes Band 16 durch das Druckkissen 25 eingezogen und im Schraubenkopf 2 jeweils durch eine Madenschraube 5 gehalten. Die Madenschraube 5 liegt auf der Schraubenachse 21 der Pedikelschraube. An den Seitenflächen des Kopfes 2 sind Kerben 24 angebracht in die ein Halter 6b (Figur 6) einfahren kann, um beim Anziehen der Madenschrauben 5 mit einem Drehschrauber 10 ein entsprechendes Gegenmoment zu bilden.

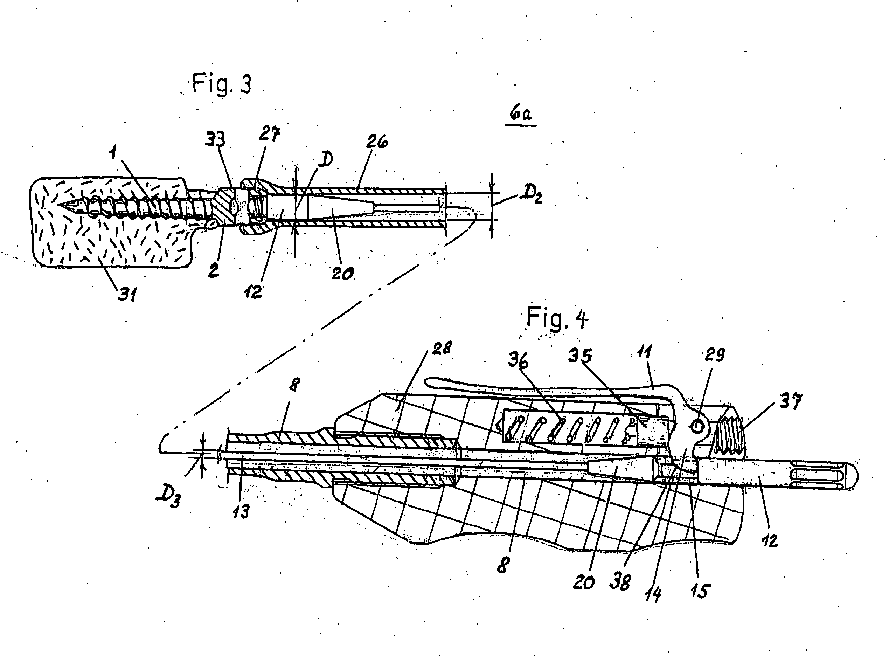

An den Figuren 3, 4, 5 und 8 wird das Ausführungsbeispiel eines ersten Halters 6a und sein Zusammenwirken am Kopf 2 einer Pedikelschraube 1 mit einem Zentrierteil 12 und mit einer als Madenschraube 5 ausgeführten Klemmschraube aufgezeigt. Der Halter 6a besteht aus einem Vorderteil 26, das als rohrförmiges Teil 8 ausgeführt ist und in einer Mündung 27 endet, welche sich auf dem Kopf 2 der Pedikelschraube 1 abstützt und die freien Stirnflächen 33 umgreift, um ein genügend grosses Drehmoment für das Eindrehen der Pedikelschraube 1 aufzubringen. Ein Zentrierteil 12 ist im Innengewinde 3 des Kopfes 2 mit einem Gewindeteil verankert, der dem Aussengewinde 9 einer als Madenschraube 5 ausgeführten Klemmschraube entspricht, jedoch wesentlich kürzer ist, um auch bei eingeschraubtem und mit einem Durchmesser D aufsitzendem Zentrierteil 12 später ein Band 16 einziehen zu können. Die Madenschraube 5 besitzt einen grössten Durchmesser D ₁ und einen Innensechskant 39 für den Drehschrauber 10. Der Innendurchmesser D ₂ des rohrförmigen Teils 8 ist nur so gross wie der eines weiteren Halters 6 b (Figur 6) gewählt, welcher als Führung beim Einbringen der Klemmschraube 5 geeignet ist.

Das Zentrierteil 12 ist daher mit seinem Durchmesser D auf den Innendurchmesser D₂ des rohrförmigen Teils 8 von beiden Haltern 6a, 6b, 6c, 6d abgestimmt, da es in beiden Haltern längs verschiebbar sein muss, um die Halter bei eingeschraubtem Zentrierteil 12 gegeneinander auswechseln zu können und andererseits bei aufgesetztem ersten Halter 6 a das Zentrierteil 12 einfahren zu können, und sicher in das Innengewinde 3 am Kopf einzuschrauben.

Das Vorderteil 26 des ersten Halters 6 a ist in einem Griffteil 28 fest verschraubt, in welchem sich die Rohrform mit Durchmesser D₂ fortsetzt. Am Zentrierteil 12, auf dessen mittleren Bereich 13 später eingegangen wird, ist im Bereich des Griffteils 28 eine umlaufende Quernut 15 angebracht, in die ein vorn gegabelter Hebel 11 hineinragt, um das eingeschraubte Zentrierteil 12 mit einer vorgegebenen Axialkraft axial zu verriegeln. Die Funktion, die dieses Feststellelement 14 ausübt, liesse sich ebenso mit einer radial einfahrenden Stellschraube verwirklichen, deren vordere Kante konisch zulaufend ausgeführt ist. Ohne die Quernut 15 würde schon eine Klemmung durch eine radial eingedrehte Schraube genügen. Im vorliegenden Fall ist der mit einer Achse 29 fixierte Hebel 11 mit einer Kontur 38 versehen, auf die ein von einer Druckfeder 36 beaufschlagter Stössel 35 presst. In der gezeichneten Lage ist das Zentrierteil 12 verriegelt. Beim Zurückdrehen des Hebels 11 gelangt der vordere Teil der Kontur 38 welcher einem Kreisbogenstück um die Achse 29 entspricht, auf den Stössel und in einen Bereich der senkrecht von der Achse 29 zum Stössel 35 steht, so dass das Zentrierteil 12 freigegeben ist und der erste Halter 6a vom Kopf 2 gelöst und abgezogen werden kann, während das Zentrierteil 12 im Kopf verbleibt. Ein Stopfen 37 verschliesst die Bohrung, welche zum Einbringen von Druckfedern 36 und Stössel 35 notwendig ist. Das Zentrierteil 12 in Figur 5 ist in seinem mittleren Bereich 13 als Biegefeder mit einem Durchmesser D₃ ausgeführt, der jeweils über ein konisches Übergangsstück 20 erreicht wird. Dieser mittlere Teil 13 kann sich beispielsweise bis zu 20° oder mehr elastisch auslenken. Die konischen Übergangsstücke 20 verhindern, dass der mittlere Teil 13 trotz Auslenkung an irgendwelchen Kanten hängen bleibt.

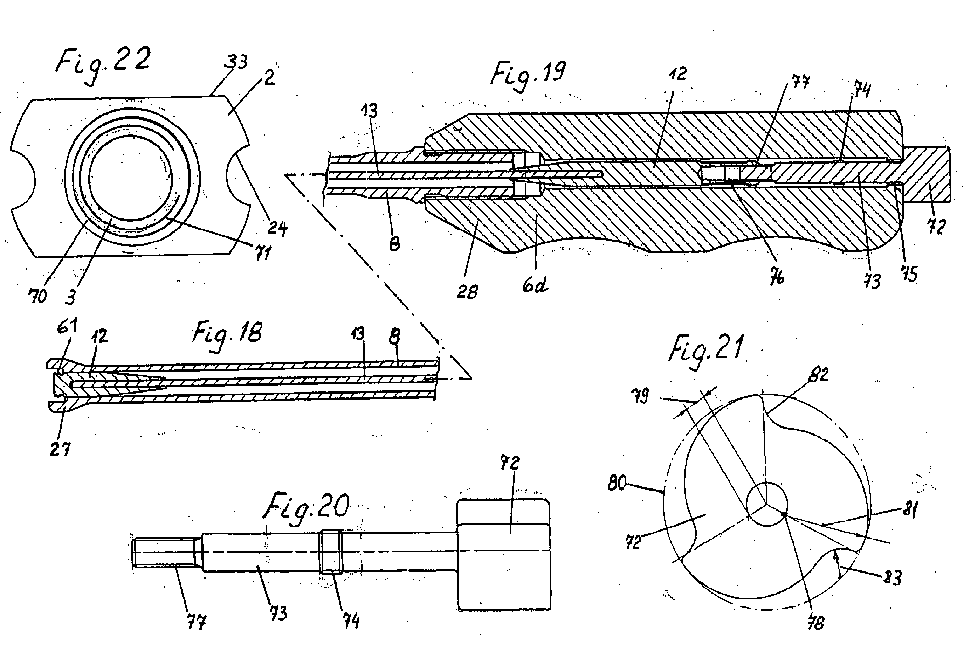

Anhand der Figuren 18 bis 21 ist ein weiterer Halter 6 d analog zum Halter 6 a in den Figuren 3 und 4 mit einem Zentrierteil 12 gezeigt. Da es sich um einen ersten Halter 6 d handelt, der als Setzinstrument verwendet wird, kann zunächst das Zentrierteil 12, welches beim späteren Entfernen des Halters stehen bleiben soll, an der Pedikelschraube angeschraubt werden und anschliessend der Halter 6 d aufgeschoben werden. In diesem Fall muss das Zentrierteil 12 nicht durch den Halter hindurch eingeschoben werden. Der Halter 6 d, der ebenfalls einen rohrförmigen Teil 8 mit Mündung 27 aufweist, besitzt einen verlängerten Griffteil 28 mit einer in der Längsachse hineinragenden Spannschraube 73, die sich mit ihrem aussen greifbaren Kopf 72 am Griffteil 28 abstützt und mit einem Spanngewinde 77 bis in ein Innengewinde 76 am Ende des Zentrierteils 12 hineinragt. Das Zentrierteil 12 besitzt am anderen Ende ein Gewinde 61 mit einer anschliessenden Schulter, mit welcher es beim Einschrauben des Gewindes 61 gegen den Kopf 2 einer Pedikelschraube verkeilt ist, während das Spanngewinde 77 ohne Verkeilung am Innengewinde 76 aufliegt und sich daher immer als erstes Gewinde löst, wenn die Spannschraube 73 zurückgedreht wird. Die Spannschraube 73 selbst ist durch die Reibung ihres Kopfes 72 gegen den Griffteil 28 blockiert. Es versteht sich, dass das Gewinde 77 der Spannschraube 73 gleichlaufend wie das Gewinde 61 des Zentrierteil 12 ausgeführt ist.

Die Spannschraube 73 hat zwei Besonderheiten, die ganz allgemein für handbetätigte Schrauben in der Technik anwendbar sind. Um einer Überbelastung der Gewinde 77, 76 beim Spannen vorzubeugen, ist der Kopf 72 so klein gehalten und mit einem asymmetrischen Profil versehen, dass nur kleine Drehmomente aufgebracht werden können und dass wegen der Asymmetrie grössere Drehmomente zum Lösen als zum Spannen von Hand aufbringbar sind.

Figur 21 zeigt ein solch asymmetrisches Profil. Für den konkreten Fall einer Spannschraube 73 mit Kopf 72, die für ein Zentrierteil 12 an einer Pedikelschraube vorgesehen ist, wird das Profil von einem Aussenkreis 80 mit Durchmesser 15 mm eingeschlossen. Das Profil wiederholt sich im Abstand von 120° und ist für eine Spannschraube 73 mit Rechts-Gewinde vorgesehen. Aus diesem Grund verlaufen die im Gegenuhrzeigersinn anstehenden Flanken, über die das Spannmoment aufgebracht werden muss, viel flacher als die im Uhrzeigersinn für das Lösen anstehenden Flanken. Wenn man von einer maximalen in radialer Richtung möglichen Greifkraft ausgeht, dann wird diese bei einem wesentlich kleineren Drehmoment im Uhrzeigersinn erreicht. Im gezeigten Beispiel mit Aussenkreis 80 mit Durchmesser 15 mm werden die im Uhrzeigersinn anstehenden Flanken durch einen Kreisbogen mit einem Radius 81 erzeugt, der tangential in den Aussenkreis 80 übergeht und sein Zentrum auf einem Immenkreis mit Radius 79 hat, der hier 1,5 mm beträgt. Die im Gegenuhrzeigersinn anstehenden Flanken werden mit einem Kreisbogen mit einem Radius 83 erzeugt, der den Aussenkreis senkrecht schneidet und einen Betrag von 2 mm aufweist, so dass im Gegenuhrzeigersinn eine Schulter 82 von etwa 2 mm vorsteht.

Grundsätzlich sind auch andere Winkelabstände als 120° möglich um zu einem ähnlichen Resultat bezüglich Drehmomentbegrenzung zu kommen.

Ein weiteres Merkmal der Spannschraube 73 verhindert, dass sich diese unvorhergesehen vom Griffteil 28 löst und beispielsweise aus dem sterilen Bereich zu Boden fällt. Am Schaft der Spannschraube 73 ist ein vorstehendes Gewinde 74 angebracht, das durch ein Gegengewinde 75 im Griffteil 28 durchgeschraubt werden muss. Bevor die Spannschraube 73 tiefer in den Griffteil hineingestossen werden kann. Wenige Gewindegänge verhindern so, dass sich die Spannschraube auf ihrem Transport von der Sterilisation in den Operationssaal oder bei mehrfacher Verwendung des Halters vom Griffteil löst.

Figur 22 und 18 zeigen eine Massnahme, die es gestattet mit einer kreisringförmigen Abstützfläche 70 in axialer Richtung am Kopf 2 einer Pedikelschraube eine Ausrichtung vom rohrförmigen Teil 8, vorzunehmen, wenn eine entsprechende Gegenfläche an der Mündung 27 der Halter 6 a und 6 d vorhanden ist. Für eine seitliche Zentrierung, das heisst für das Zusammenfallen von den Längsachsen von der Pedikelschraube 1 und dem rohrförmigen Teil 8 sorgen die über die Abstützflächen 70 vorstehenden Backen der Mündung 27. Die Pedikelschraube kann somit trotz der kugelförmigen Teilflächen am Kopf 2 nicht wie ein Gelenk in der Mündung 27 bewegt werden. Eine solche Anordnung ist nicht nur für handbetätigte Setzeinrichtungen entsprechend den Haltern 6 a und 6 d von Vorteil, sondern kann auch bei Setzwerkzeugen von Navigationssystemen (CAS, computer assisted surgery) verwendet werden, wenn die Lage der Spitze einer Pedikelschraube zu einem Halter mit erfassbarer Referenzlage genau eingehalten werden soll, immer unter der Voraussetzung, dass dieser Halter ein Zentrierteil 12 besitzt, welches im Kopf der Pedikelschraube stehen bleibt, wenn der Halter von der eingedrehten Pedikelschraube gelöst und abgezogen wird. Ein solches Zentrierteil würde dann im Sinne der Erfindung die anschliessenden manuellen Operationsschritte mit passenden weiteren Haltern begünstigen.

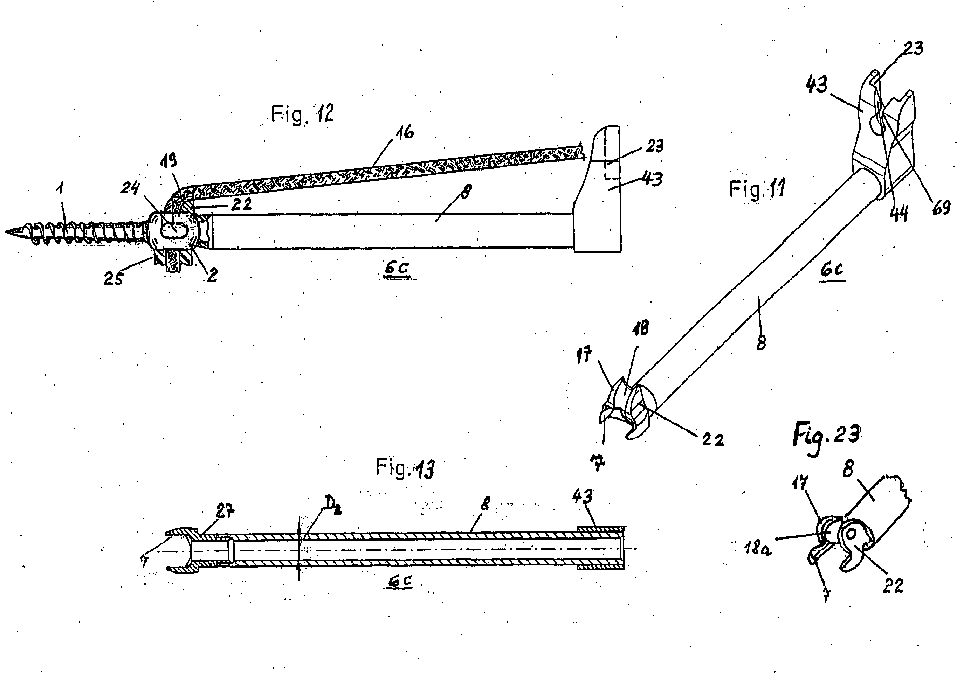

Mit den Figuren 6, 7, 8 und 9 wird die Funktion eines zweiten Halters 6 b beschrieben, der nach dem Ausfahren des ersten Halters 6 a oder 6 d auf den Kopf 2 der 1. Pedikelschraube 1 aufschiebbar ist und sich dabei am Zentrierteil 12 orientiert. Vorsprünge 7 fahren dabei formschlüssig in Kerben 24 am Kopf 2 der Pedikelschrauben ein. Ein Mundstück 27 ist bei diesem Halter als separates Teil aus einem hochwertigen, verschleissfesten Stahl hergestellt und anschliessend mit dem rohrförmigen Teil 8 verbunden worden, welches durch einen quergestellten Griff 40 führt, um ein Gegemoment beim Anziehen der Madenschraube 5 zu erzeugen. Der Innendurchmesser D ₂ des rohrförmigen Teils 8 ist kleiner als das 1,3-fache oder noch besser kleiner als das 1,1-fache des Aussendurchmessers D₁ der Madenschraube 5, um diese nach dem Abziehen des Zentrierteils 12 geführt bis an das Innengewinde 3 im Kopf zu stossen. Der Drehschrauber 10 in Figur 9 besitzt einen Sechskant der in den Innensechskant 39 der Madenschaube 5 passt. Der Aussendurchmesser D₄ des Drehschraubers 10 ist so gewählt, dass dieser im rohrförmigen Teil 8 mit dem Innendurchmesser D₂ geführt ist und den Innensechskant 39 der Madenschraube 5 findet. Der vordere Teil des Drehschraubers 10 mit dem Durchmesser D ₄ ist so lang ausgeführt, dass er bei Haltern 6b und 6c verwendbar ist um mit seinem Handgriff 40 eingesetzte Madenschrauben 5 festzuziehen.

Mit den Figuren 11, 12 und 13 ist ein weiterer Halter 6c gezeigt, welcher auf der zweiten Pedikelschraube über ein Zentrierelement 12 aufschiebbar ist. Vorgängig wurde die zweite Pedikelschraube mit dem ersten Halter 6 a oder 6 d gesetzt, dieser wurde anschliessend abgezogen und ein Zentrierelement 12 blieb stehen. Anschliessend wurde mit dem in Figur 10 gezeigten Spreizwerkzeug 42 der Abstand zwischen erster und zweiter Pedikelschaube unter einer bestimmten Distraktionskraft festgestellt und ein passendes Druckkissen 25 auf das Band 16 aufgezogen und das Band bei immer noch aufgesetztem Zentrierteil 12 durch den Kopf 2 der zweiten Pedikelschraube gezogen.

Erst jetzt wird der weitere Halter 6 c über das Zentrierteil 12 auf die zweite Pedikelschraube aufgeschoben bis seine Vorsprünge 7 formschlüssig in die Kerben 24 eingreifen und dann wird das Zentrierteil 12 entfernt. Eine Madenschraube 5 kann in den rohrförmigen Teil 8 eingeschoben werden und mit dem Drehschrauber 10 an den Kopf 2 der Pedikelschraube angesetzt werden.

Der weitere Halter 6 c besitzt ebenfalls eine separat hergestellte Mündung 27, an die ein Schuh 17 angeformt ist, welcher bei aufgesetztem Halter eine Durchgangsbohrung durch Kopf 2 mit einem Umlenkbogen 18 um annähernd 90 ° fortsetzt, wobei der Umlenkbogen 18 stufenlos mit seinem Grund 19 an der Durchgangsbohrung ansetzt. Der Umlenkbogen ist mit einem Krümmungsradius von mehr als 3 mm ausgeführt, damit auch bei einem steifen durch die Durchgangsbohrung gezogenen Band 16 nicht zuviel Reibung durch die Umlenkung entsteht. In einer Variante (siehe Figur 23) ist der Umlenkbogen 18 durch einen beweglichen Umlenkbogen, das heisst durch eine Rolle 18a ersetzt worden, welche in dem Schuh 17 in seitlich vorstehenden Backen gelagert ist.

Der obere Teil des Halters 6 c ist als auskragender Arm 43 ausgeführt, welcher Kupplungsflächen 23 für einen zangenförmigen Bandspanner (nicht gezeigt) aufweist. Derartige Bandspanner sind separate, zangenähnliche Werkzeuge mit zwei sich zueinander aufweitenden Schenkeln von denen sich einer beispielsweise am Kopf 2 der Pedikelschraube abstützt, während der andere das Band greift und am ersten Schenkel vorbei zieht. Bandspanner werden als Werkzeuge zu dem vorher erwähnten Produkt DYNESYS ® der Firma Centerpulse Orthopedics Ltd verkauft. Ein solcher Bandspanner wird mit dem nicht greifenden Schenkel auf der Kupplungsfläche 23 angesetzt, nachdem das Band 16 durch den greifenden Schenkel gepackt wurde. Die Kupplungsfläche 23 sichert den nicht greifenden Schenkel gegen Drehung um den rohrförmigen Teil 8 und stützt ihn gleichzeitig in der Richtung zu dem Umlenkbogen 18 ab.

Ein Operateur kann daher mit der einen Hand den aufgesetzten Bandspanner betätigen und ein Gegenmoment über den Halter 6c und dessen Vorsprünge 7 an dem Kopf 2 der Pedikelschraube erzeugen, während er mit der anderen Hand den Drehschrauber 10 betätigen kann, um das Band festzusetzen, wenn eine vorgegebene Zugspannung im Band 16 erreicht ist.

In der Kupplungsfläche 23 ist zum Umlenkbogen hin ein Schlitz 44 angebracht, durch den das Band 16 seitlich eingebracht wird, wenn der Bandspanner angesetzt wird. Dabei ist es vorteilhaft, wenn der Schlitz 44 über einen Engpass 69 in eine grössere Bohrung übergeht und der Engpass 69 so bemessen ist, dass ein Band seitlich unter Krafteinwirkung in die Bohrung gestossen werden muss, damit es in der Bohrung gefangen ist und aus dem Operationsfeld herausgeführt ist, ohne ungewollt wieder in das Operationsfeld zurückfallen kann.

In Figur 10 ist ein Spreizinstrument 42 gezeigt, bei dem sich zwei Hebel 47, 48 wie eine Zange in einem Drehpunkt 53 kreuzen. An einem der Hebel 47 ist mit Schrauben 57 ein Querbalken 49 angebracht, welcher den anderen Hebel 48 in einem Bogen kreuzt und in einem Handgriff 50 endet. Der erste Hebel setzt sich in zwei Teilen nach aussen fort, in einem starren Zeigerteil 52 und in einer aufgesetzten Biegefeder 51, die mit einem Daumengriff 56 über den Bogen des Querbalkens 49 vorsteht.

Wenn man den Handgriff 50 in der gezeichneten Lage mir vier Fingern der rechten Hand umfasst und den Daumen in den Daumengriff 56 legt, kann man die Vorrichtung durch Anziehen des Daumens spreizen.

Die Spitze des ersten Hebels 47 ist gabelförmig. Diese Gabel 46 liegt am Kopf der ersten Pedikelschraube an und zentriert sich an dem austretenden Band 16. Die Spitze des anderen Hebels 48 endet in einer seitlich wegstehenden Halbkugel 45, welche sich an der Durchgangsbohrung der zweiten Pedikelschraube zentriert. Wenn nun durch Anziehen des Daumens eine Spreizkraft aufgebracht wird, deformiert sich die Biegefeder 51 zum Handgriff 50 hin, während der starre Zeigerteil 52 nur um soviel am Bogen des Querbalkens 49 weiterwandert, wie sich die Spitzen der Hebel 47, 48 auseinander bewegen.

Auf dem bogenförmigen Teil des Querbalkens 49 ist eine erste Skala 54 angebracht, auf der der äussere Abstand der Spitzen der Hebel 47, 48 abgelesen werden kann.

Mit dem Daumengriff 56 ist eine weitere, zweite Skala 55 verbunden, welche die Bewegung der Biegefeder 51 mitmacht und relativ zum Zeigerteil 52 anzeigt unter welcher Vorspannkraft die beiden Spitzen auseinander gedrückt werden. Der Zweck des Instruments besteht darin, dass es einhändig bedienbar ist, und dass für eine zwischen den Wirbeln vorgegebene Vorspannkraft die Länge festgestellt wird, welche eine Referenzgrösse für ein später einzusetzendes Druckkissen 25 bildet. Entsprechend den elastischen Eigenschaften des Materials für das Druckkissen 25 muss dieses im unverspannten Zustand im Hinblick auf die Referenzgrösse so zugeschnitten werden, dass es nach dem Einbau und Spannen des Bandes eine vorgesehene Länge einnimmt.

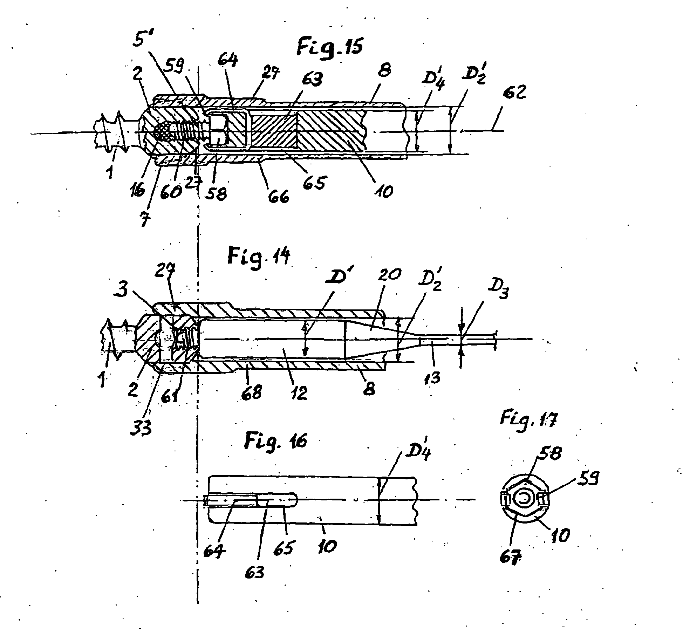

Eine weitere Anwendung der Erfindung lässt sich gemäss den Figuren 14, 15, 16, 17 auf weitere Ausführungen von Instrumenten übertragen, die als Klemmschraube eine Schraube 5' mit einem vorstehenden Schraubenkopf 58 beispielsweise einem Sechskant vorsehen. In einem Halter 66 gemäss Figur 15 ist für den Innendurchmesser D₂' eines rohrförmigen Teils 8, der Aussendurchmesser D ₄' eines Drehschraubers 10 massgebend, welcher an seinem passenden Innensechskant 67 eine Greifeinrichtung 59 für einen Schraubenkopf 58 besitzt, um die Sechskantschraube 5' ausgerichtet auf die Achse 62 des Drehschraubers 10 zu halten. Der in seinem Innendurchmesser D₂' nur geringfügig, beispielsweise um weniger als das 1,2-fache, grössere rohrförmige Teil 8 führt dann indirekt über den Drehschrauber 10 die Klemmschraube mit ihrem Gewinde 60 bis an das Innengewinde 3 im Kopf 2 der Pedikelschraube 1 heran. Der Halter 66 stützt sich mit seiner Mündung 27 auf dem Kopf 2 der Pedikelschraube ab und greift mit Vorsprüngen 7 formschlüssig in Kerben ein, um ein Gegenmoment beim Eindrehen der Sechskantschraube 5' zu bilden, welche das eingezogene Band 16 festsetzt. Der Drehschrauber 10 umfasst mit einem Innensechskant 67 den Schraubenkopf 58, wobei mit einer Feder 64, die gegenüberliegend über den unteren Rand des Schraubenkopfes 58 hinweggreift, die Schraube 5' in den Innensechskant 67 gepresst wird.

Wegen der beengten Platzverhältnisse ist die Feder C-förmig ausgeführt und von hinten durch eine Keilnut 65 im Schaft des Drehschraubers 10 aufgeschoben worden und durch einen nachträglichen eingeschobenen Keil 63 gesichert worden. Die Schenkel der Feder 64 bilden an ihrem Ende einen Vorsprung, der beim Einsetzen des Schraubenkopfes 58 zurückfedert und diesen an seiner Unterkante hält. Die Schenkel selbst sind jeweils in einer Nut eingelassen, damit sie nicht über den Durchmesser D₄ des Drehschraubers vorstehen.

In Figur 14 ist ein zu Figur 15 passender Halter 68 mit einem Zentrierteil 12 gezeigt, dessen Durchmesser D' geringfügig kleiner als der Durchmesser D₂' der Halter 66, 68 ist, damit diese ihre formschlüssigen Gegenflächen am Kopf 2 der Pedikelschraube 1 finden.

Das Zentrierteil 12 ist mit einem Gewinde 61 im Innengewinde 3 am Kopf 2 einer Pedikelschraube 1 verankert. Es besitzt konische Übergänge 20 zu einem mittleren Teil 13, welcher biegeelastisch mit einem kleineren Durchmesser D₃ ausgeführt ist. Der Halter 68 ist mit seiner Mündung 27 an die abgeflachten Stirnflächen 33 des Kopfes 2 angepasst.

Zu den beiden Haltern in Figur 14 und 15 gibt es einen weiteren Halter, der ein rohrförmiges Teil wie der Halter in Figur 15 besitzt und ebenfalls den Schuh 17 und eine Umlenkvorrichtung 22 wie der Halter 6 c (Fig.8) und einen Arm 43 für einen Bandspanner aufweist. Vom Instrumentensatz gemäss Figur 15 bis 17 lässt sich allgemein sagen, dass die Zentrierteile 12 einen grössten Durchmesser D' besitzen der kleiner als der Durchmesser D₂' des rohrförmigen Teils 8 ist. Andere Dimensionen wie beispielsweise Wandstärken vom rohrförmigen Teil 8 sowie weitere Details und Längenabmessungen der Instrumente können dem Instrumentensatz der Figuren 3 bis 9, 11 bis 13 und Figur 21 entsprechen.

### Teileliste

- 1: Pedikelschraube
- 2: Kopf
- 3: Innengewinde
- 4: Verbindungsteil
- 5: Madenschraube
- 5': Schraube
- 6: Halter
- 6a: erster Halter
- 6b: zweiter Halter
- 6c: weiterer Halter
- 6d: Halter
- 7: Vorsprung
- 8: rohrförmiger Teil
- 9: Aussengewinde
- 10: Drehschrauber
- 11: Hebel
- 12: Zentrierteil
- 13: mittlerer Bereich
- 14: Feststellelement
- 15: Quernut
- 16: Band
- 17: Schuh
- 18: Umlenkbogen
- 18a: Umlenkrolle
- 19: Grund
- 20: Konus
- 21: Schraubenachse
- 22: Umlenkvorrichtung
- 23: Kupplungsfläche
- 24: Kerbe
- 25: Druckkissen
- 26: Vorderteil
- 27: Mündung
- 28: Griffteil
- 29: Achse
- 30: Stützelement
- 31: Rückenwirbel
- 32: Rückenwirbel
- 33: Stirnfläche
- 34: Bandscheibe
- 35: Stössel
- 36: Feder
- 37: Stopfen
- 38: Kontur
- 39: Innensechskant
- 40: Griff
- 41: Griff
- 42: Spreizinstrument
- 43: Arm
- 44: Schlitz
- 45: Halbkugel
- 46: Gabel
- 47: Hebel
- 48: Hebel
- 49: Querbalken
- 50: Handgriff
- 51: Biegefeder
- 52: Zeigerteil
- 53: Drehpunkt
- 54: 1. Skala
- 55: 2. Skala
- 56: Daumengriff
- 57: Schrauben
- 58: Schraubenkopf
- 59: Greifvorrichtung
- 60: Gewinde
- 61: Gewinde
- 62: Achse
- 63: Keil
- 64: Feder
- 65: Keilnut
- 66: Halter
- 67: Innensechskant
- 68: Halter
- 69: Engpass
- 70: Absützfläche
- 71: Ansenkung
- 72: Kopf
- 73: Spannschraube
- 74: Gewinde
- 75: Gegengewinde
- 76: Innengewinde
- 77: Spanngewinde
- 78: Innenkreis
- 79: Radius
- 80: Aussenkreis
- 81: Radius
- 82: Schulter
- 83: Schmiegeradius

- D: Durchmesser

- D₁: Durchmesser Klemmschraube aussen

- D₂: Durchmesser rohrf. Teil innen

- D₃: Durchmesser mittlerer Teil Zentrierteil

- D₄: Durchmesser Drehschrauber

## Patentansprüche

1. Instrumentensystem mit Pedikelschrauben (1), Klemmschrauben (5, 5'), insbesondere Madenschrauben (5), einem Verbindungsteil (4), einem Drehschrauber (10), wenigstens zwei Haltern (6a, 6b; 6c, 6d) und einem Zentrierteil (12), wobei die Pedikelschrauben (1) jeweils mit einem Innengewinde (3) in ihrem Kopf (2) versehen sind, das in der Richtung ihrer Schraubenachse (21) eine der Klemmschrauben (5, 5') mit Aussendurchmesser D₁ aufnimmt, um das Verbindungsteil (4), welches quer zur Schraubenachse (21) in den Kopf (2) hineinragt, festzuklemmen, wobei die Halter (6a, 6b; 6c, 6d) mit Vorsprüngen (7) jeweils formschlüssig am Kopf (2) der Pedikelschrauben (1) eingreifen können und der Drehschrauber (10) mit Durchmesser D₄ an die Klemmschrauben (5, 5') ansetzbar ist, wobei die Halter (6a, 6b; 6c, 6d) jeweils mit einem rohrförmigen Teil (8) versehen und mit dem rohrförmigen Teil (8) auf dem Kopf (2) der Pedikelschrauben (1) abstützbar sind, wobei statt der Klemmschraube (5, 5') das Zentrierteil (12) einsetzbar ist, welches ebenfalls in das im Kopf (2) ausgebildete Innengewinde (3) der Pedikelschrauben (1) einschraubbar ist, bei aufgesetztem Halter (6 a, 6 d) greifbar ist und in seinem Aussendurchmesser D dem Durchmesser D₁ der Klemmschrauben (5, 5') entspricht, um den gleichen Halter (6 a, 6 d) oder andere Halter (6 b, 6 c) abziehen und beliebig oft wieder an formschlüssige Gegenflächen der Pedikelschrauben (1) heranführen zu können, und wobei der rohrförmige Teil (8) der Halter (6a, 6b; 6c, 6d) jeweils einen Innendurchmesser D₂ aufweist, der nur wenig grösser als der Durchmesser D₁ der Klemmschrauben (5, 5') ist, um die jeweilige Klemmschraube (5, 5') mittels des Drehschraubers (10) oder das Zentrierteil (12) durch das rohrförmige Teil (8) geführt an den Kopf (2) der Pedikelschraube (1) anzusetzen, so dass deren Gewinde mit dem Innengewinde (3) im Kopf (2) der Pedikelschraube (1) unverkantet greifen, **dadurch gekennzeichnet, dass** das Zentrierteil (12) einen in Längsrichtung mittleren Bereich (13) aufweist, welcher elastisch bis um einen Winkel von 20 ° oder mehr von der Längsachse weg auslenkbar ist.

2. Instrumentensystem nach Anspruch 1, **dadurch gekennzeichnet, dass** der Innendurchmesser D₂ weniger als das 1,3-fache des Durchmessers D₁ beträgt.

3. Instrumentensystem nach Anspruch 1, **dadurch gekennzeichnet, dass** der Innendurchmesser D₂ weniger als das 1,1-fache des Durchmessers D₁ beträgt.

4. Instrumentensystem nach einem der Ansprüche 1 bis 3, **dadurch gekennzeichnet, dass** das Zentrierteil (12) in seinem mittleren Bereich (13) einen geringeren Durchmesser D₃ aufweist, um als Biegefeder eine vorgesehene Auslenkung zuzulassen.

5. Instrumentensystem nach einem der vorhergehenden Ansprüche, **dadurch gekennzeichnet, dass** das Zentrierteil (12) im wesentlichen kreisförmige Querschnitte und in Längsrichtung im Anschluss an den mittleren Bereich (13) Mantellinien mit sanften, stufenlosen Übergängen aufweist.

6. Instrumentensystem nach Anspruch 1, **dadurch gekennzeichnet, dass** ein rohrförmiger Halter (6a, 6d) mit einem Zentrierteil (12) als Setzvorrichtung für Pedikelschrauben (1) ausgebildet ist, in welchem der aufgesetzte Halter (6a, 6d) ein in axialer Richtung blockierendes Feststellelement (14, 73) zum Zentrierteil (12) aufweist, welches seinerseits im Kopf (2) der Pedikelschraube (1) eingeschraubt ist.

7. Instrumentensystem nach Anspruch 6, **dadurch gekennzeichnet, dass** der aufgesetzte Halter (6d) ein Feststellelement in Form einer Spannschraube (73) aufweist, welche sich mit ihrem Kopf (72) auf dem Halter (6 d) abstützt und in axialer Richtung in ein Innengewinde (76) am hinteren Teil des Zentrierteils (12) eingreift.

8. Instrumentensystem nach Anspruch 6, **dadurch gekennzeichnet, dass** das Feststellelement (14) als Hebel (11) auf eine Quernut (15) des Zentrierteils (12) wirkt.

9. Instrumentensystem nach einem der Ansprüche 1 bis 3, **dadurch gekennzeichnet, dass** der rohrförmige Teil (8) als Umlenkvorrichtung (22) für ein durch den Schraubenkopf (2) durchgezogenes Band (16) einen vorstehenden Schuh (17) mit einem Umlenkbogen (18) und am anderen Ende des rohrförmigen Teils (8) bezüglich Drehung eine formschlüssige Kupplungsfläche (23) für einen Bandspanner aufweist, welche den Bandspanner in Richtung zu der Pedikelschraube (1) abstützt.

10. Instrumentensystem nach Anspruch 9, **dadurch gekennzeichnet, dass** der Umlenkbogen (18) längs seinem Grund (19) einen Krümmungsradius grösser 3 mm aufweist.

11. Instrumentensystem nach Anspruch 9, **dadurch gekennzeichnet, dass** der Schuh (17) statt einem Umlenkbogen eine Umlenkrolle (18 a) aufweist.

## Claims

1. An instrument system having pedicle screws (1), clamping screws (5, 5'), in particular grub screws (5), a connection part (4), a screwdriver (10), at least two holders (6a, 6b; 6c, 6d) and a centring part (12), wherein the pedicle screws (1) are each provided with an internal thread (3) in their head (2), said thread accepting one of the clamping screws (5, 5')) with an external diameter D₁ in the direction of its screw axis (21) to fixedly clamp the connection part (4) which projects transversely to the screw axis (21) into the head (2), with projections (7) of the holders (6a, 6b; 6c, 6d) each being able to engage in a shape matched manner at the head (2) of the pedicle screws (1) and the screwdriver (10) with a diameter D₄ being able to be positioned at the clamping screws (5, 5'), wherein the holders (6a, 6b, 6c, 6d) are each provided with a tubular part (8), and are able to be supported with the tubular part (8) at the head (2) of the pedicle screws (1), wherein the centring part (12) is insertable instead of the clamping screw (5, 5'), said centring part likewise being able to be screwed into the internal thread (3) formed in the head (2), being able to be gripped when the holder (6a, 6d) is set in place and corresponding in its external diameter D to the diameter D₁ of the clamping screws (5, 5') in order to be able to pull off the same holder (6a, 6d) or other holders (6b, 6c) and to be able to guide them as often as desired to shape matched mating surfaces of the pedicle screws (1), and wherein the tubular part (8) of the holders (6a, 6b; 6c, 6d) respectively has an internal diameter D₂ which is only a little larger than the diameter D₁ of the clamping screws (5, 5') in order to position the respective clamping screw (5, 5') by means of the screwdriver (10) or the centring part (12) guided through the tubular part (8) at the head (2) of the pedicle screw (1) such that its threads engage with the internal thread (3) in the head (2) of the pedicle screw (1) in a non-canted manner, **characterised in that** the centring part (12) has a central region (13) in the longitudinal direction which can be resiliently deflected up to an angle of 20° or more away from the longitudinal axis.

2. An instrument system in accordance with claim 1, **characterised in that** the internal diameter D₂ amounts to less than 1.3 times the diameter D₁.

3. An instrument system in accordance with claim 1, **characterised in that** the internal diameter D₂ amounts to less than 1.1 times the diameter D₁.

4. An instrument system in accordance with any one of the claims 1 to 3, **characterised in that** the centring part (12) has a lower diameter D₃ in its central region (13) in order to allow an envisaged deflection as a flexural spring.

5. An instrument system in accordance with any one of the preceding claims, **characterised in that** the centring part (12) has substantially circular cross-sections and jacket lines with gentle, stepless transitions in the longitudinal direction following the central region (13).

6. An instrument system in accordance with claim 1, **characterised in that** a tubular holder (6a) is formed with a centring part (12) as a positioning apparatus for pedicle screws (1) in which the mounted holder (6a, 6d) has a fixing element (14) with respect to the centring part (12) which blocks in the axial direction, said centring part (12) in turn being screwed into the head (2) of the pedicle screw (1).

7. An instrument system in accordance with claim 6, **characterised in that** the mounted holder (6d) has a fixing element in the form of a clamping screw (73) which is supported at its head (72) on the holder (6d) and engages in the axial direction into an internal thread (76) at the rear part of the centring part (12).

8. An instrument system in accordance with claim 6, **characterised in that** the fixing element (14) acts as a lever (11) on a transverse groove (15) of the centring element (12).

9. An instrument system in accordance with any one of claims 1 to 3, **characterised in that** the tubular part (8) has, as a deflection apparatus (22) for a band or cable (16) pulled through the screw head (2,) a projecting shoe (17) with a deflection arc (18) and, at the other end of the tubular part (8), with respect to rotation, a shape matched coupling surface (23) for a band or cable tensioner which supports the band or cable tensioner in the direction towards the pedicle screw (1).

10. An instrument system in accordance with claim 9, **characterised in that** the deflection arc (18) has a radius of curvature larger than 3 mm along its base (19).

11. An instrument system in accordance with claim 9, **characterised in that** the shoe (17) has a deflection roller (18a) instead of a deflection arc.

## Revendications

1. Ensemble d'instruments comprenant des vis pédiculaires (1), des vis de coincement (5, 5'), en particulier des vis sans tête (5), une partie de liaison (4), un tournevis (10), au moins deux éléments de maintien (6a, 6b ; 6c, 6d) et une partie de centrage (12), les vis pédiculaires (1) étant pourvues chacune d'un taraudage (3) dans leur tête (2), qui reçoit dans la direction de son axe de vis (21) l'une des vis de coincement (5, 5') avec un diamètre extérieur D₁ pour coincer la partie de liaison (4), dépassant dans la tête (2) transversalement à l'axe de vis (21), les éléments de maintien (6a, 6b ; 6c, 6d) pouvant s'engager par des saillies (7) chacun en coopération de formes sur la tête (2) des vis pédiculaires (1) et le tournevis (10) de diamètre D₄ pouvant être appliqué aux vis de coincement (5, 5'), les éléments de maintien (6a, 6b ; 6c, 6d) étant pourvus chacun d'une partie tubulaire (8) et pouvant prendre appui par la partie tubulaire (8) sur la tête (2) des vis pédiculaires (1), la partie de centrage (12) pouvant être utilisée à la place de la vis de coincement (5, 5') et être vissée également dans le taraudage (3) réalisé dans la tête (2) des vis pédiculaires (1), être saisie lorsque l'élément de maintien (6a, 6d) est rapporté, et correspondant par son diamètre extérieur D au diamètre D₁ des vis de coincement (5, 5'), afin de permettre de retirer le même élément de maintien (6a, 6d) ou d'autres éléments de maintien (6b, 6c) et de les rapprocher autant de fois que désiré vers des contre-surfaces, en coopération de formes, des vis pédiculaires (1), et la partie tubulaire (8) des éléments de maintien (6a, 6b ; 6c, 6d) présentant chacune un diamètre intérieur D₂ qui n'est que peu supérieur au diamètre D₁ des vis de coincement (5, 5'), afin de rapporter sur la tête (2) de la vis pédiculaire (1) la vis de coincement respective (5, 5') au moyen du tournevis (10) ou de la partie de centrage (12), en étant guidée par la partie tubulaire (8), de sorte que son filetage vient en engagement sans blocage avec le taraudage (3) dans la tête (2) de la vis pédiculaire (1),
**caractérisé en ce que** la partie de centrage (12) comprend une zone (13) médiane dans la direction longitudinale qui est susceptible d'être déviée élastiquement sous un angle de 20° ou plus en éloignement de l'axe longitudinal.

2. Système d'instruments selon la revendication 1, **caractérisé en ce que** le diamètre intérieur D₂ est inférieur à 1,3 fois le diamètre D₁.

3. Système d'instruments selon la revendication 1, **caractérisé en ce que** le diamètre intérieur D₂ est inférieur à 1,1 fois le diamètre D₁.

4. Système d'instruments selon l'une des revendications 1 à 3, **caractérisé en ce que** la partie de centrage (12) présente un diamètre D₃ plus petit dans sa zone médiane (13), afin de permettre, à titre de ressort de flexion, une déviation prédéterminée.

5. Système d'instruments selon l'une des revendications précédentes, **caractérisé en ce que** la partie de centrage (12) comprend des sections transversales sensiblement circulaires et, dans la direction longitudinale, suite à la zone médiane (13), des génératrices à transitions douces sans gradins.

6. Système d'instruments selon la revendication 1, **caractérisé en ce qu'**un élément de maintien tubulaire (6a, 6d) est réalisé avec une partie de centrage (12) à titre de dispositif de pose pour des vis pédiculaires (1), dans laquelle l'élément de maintien rapporté (6a, 6d) comprend un élément d'immobilisation (14, 73) par rapport à la partie de centrage (12), qui se bloque dans la direction axiale et qui est vissé à son tour dans la tête (2) de la vis pédiculaire (1).

7. Système d'instruments selon la revendication 6, **caractérisé en ce que** l'élément de maintien rapporté (6d) comprend un élément d'immobilisation sous la forme d'une vis de serrage (73) qui prend appui par sa tête (72) sur l'élément de maintien (6d) et qui s'engage, dans la direction axiale, dans un taraudage (76) sur la portion arrière de la partie de centrage (12).

8. Système d'instruments selon la revendication 6, **caractérisé en ce que** l'élément d'immobilisation (14) agit en tant que levier (11) sur une gorge transversale (15) de la partie de centrage (12).

9. Système d'instruments selon l'une des revendications 1 à 3, **caractérisé en ce que** la partie tubulaire (8) comprend, en tant que dispositif de déflexion (22) pour une bande (16) tirée à travers la tête de vis (2), un patin en saillie (17) pourvu d'un coude de déflexion (18), et elle comprend à l'autre extrémité de la partie tubulaire (8), une surface d'accouplement (23) en coopération de formes par rapport à la rotation destinée à un tendeur de bande, surface qui supporte le tendeur de bande dans la direction vers la vis pédiculaire (1).

10. Système d'instruments selon la revendication 9, **caractérisé en ce que** le coude de déflexion (18) comprend le long de son fond (19) un rayon de courbure supérieur à 3 mm.

11. Système d'instruments selon la revendication 9, **caractérisé en ce que** le patin (17) comprend un galet de déflexion (18a), à la place d'un coude de déflexion.
